# EUROPEAN PATENT APPLICATION

(11) **EP 3 910 340 A1**
(43) Date of publication of application: **17.11.2021**
(21) Application number: 20738230.0
(22) Date of filing: 13.01.2020
(51) Int. Cl.: G01N 33/574, G01N 33/60, A61K 51/04, A61P 35/00

(54) **COMPOSITION FOR TARGETING MEDULLARY THYROID CANCER**

(30) Priority: 11.01.2019 KR 20190003836; 10.01.2020 KR 20200003665
(71) Applicant: University-Industry Foundation, Yonsei University, Seoul 03722 (KR)
(72) Inventor: LEE, Eun Jig, Seoul 04093 (KR); KU, Cheol Ryong, Seoul 04422 (KR)
(74) Representative: Plasseraud IP
(86) International application number: PCT/KR2020/000582
(87) International publication number: WO 2020/145776

(57) **Abstract**

The present invention relates to a composition for targeting medullary thyroid cancer, the composition comprising a diagnostic radionuclide-labeled ligand of the olfactory receptor OR51E2. The composition is internalized into parafollicular C cells by the olfactory receptor OR51E2 that is expressed on the parafollicular C cells of the thyroid gland and as such, can be advantageously used for diagnosing parafollicular C cell-derived medullary thyroid cancer and identifying whether or not parafollicular C cell-derived medullary thyroid cancer metastases are. In addition, a pharmaceutical composition comprising an acetate-associated therapeutic radionuclide for treatment of medullary thyroid cancer can be used for treatment of medullary thyroid cancer because the composition is internalized into cancer cells through the binding of the acetate to the olfactory receptor OR51E2.

## Description

### Technical Field

The present invention relates to a composition for targeting medullary thyroid cancer and a pharmaceutical composition for treating medullary thyroid cancer using the expression of the olfactory receptor OR51E2 on parafollicular C-cells from which medullary thyroid cancer originates.

### Background Art

Lumps in the thyroid gland are called thyroid nodules, which are largely divided into benignancy and malignancy. Among these, malignant nodules are called thyroid cancer, and if the thyroid cancer is left untreated, the cancer can increase and invade the surrounding tissues, or cause lymph node metastases or distant metastases, and in severe cases, life can be lost. The thyroid cancer can be divided into papillary thyroid cancer, follicular thyroid cancer, poorly differentiated thyroid cancer and undifferentiated thyroid cancer (anaplastic thyroid cancer), which originate from follicular cells, depending on the type of cell from which it originated and the maturity of the cell. The thyroid cancer originating from non-follicular cells includes medullary cancer, lymphoma and metastatic cancer, and the like.

Among the types of thyroid cancer, medullary thyroid cancer accounts for less than 1% of the entire thyroid cancer, and occurs in parafollicular C-cells that secrete calcitonin regulating the amount of calcium in the body. The medullary thyroid cancer is often multiple, is relatively prone to metastasis and does not work well with radioactive iodine treatment after surgery, so that the fundamental treatment method is surgical removal of the thyroid gland. In most patients with medullary thyroid cancer, blood levels of calcitonin and CEA (carcino embryonic antigen) increase, so that the blood levels of these two factors are utilized as factors related to prognosis and recurrence of medullary thyroid cancer. However, since the two factors increase after formation of medullary thyroid cancer, it is difficult to diagnose medullary thyroid cancer early, and accordingly, there is a disadvantage that the treatment of medullary thyroid cancer is also delayed.

As a result of the present inventors' efforts to discover targeting agents for medullary thyroid cancer, the present invention was accomplished by confirming that olfactory receptors OR51E1 and OR51E2 were highly expressed in parafollicular C-cells of the thyroid gland, and confirming that these could be used for diagnosis, target and treatment of medullary thyroid cancer.

### Disclosure

### Technical Problem

It is an object of the present invention to provide a composition for targeting medullary thyroid cancer comprising a diagnostic radionuclide-labeled ligand of the olfactory receptor OR51E2.

It is another object of the present invention to provide a method for diagnosing medullary thyroid cancer and a method for identifying metastasis of medullary thyroid cancer using the composition for targeting medullary thyroid cancer.

It is another object of the present invention to provide a pharmaceutical composition for treating medullary thyroid cancer comprising acetate and a therapeutic radionuclide associated therewith as active ingredients, and a method for treating medullary thyroid cancer using the same.

### Technical Solution

In order to achieve the above objects, one aspect of the present invention provides a composition for targeting medullary thyroid cancer comprising a diagnostic radionuclide-labeled ligand of the olfactory receptor OR51E2.

The term "medullary thyroid cancer" as used herein is a thyroid cancer derived from parafollicular C-cells that secrete calcitonin, which has a low incidence rate, but belongs to the poor prognosis among thyroid cancers, because it is often multiple and shows metastasis relatively well. In addition, since radioactive iodine treatment does not work well after surgery, the fundamental treatment method is to remove the thyroid gland by surgical operation.

The term "olfactory receptor (OR)" as used herein is a receptor that selectively binds to odor substances, where humans have about 390 olfactory receptors. Olfactory nerve signals are generated in olfactory nerve cells by the selective coupling between the ordor substances and the olfactory receptors, and the generated olfactory nerve signals are detected by the brain to recognize the ordor. However, according to recent studies, olfactory receptors are also expressed in skins, muscles, kidneys, prostate, and the like (ectopic expression) besides the nose, and studies on various functions of the olfactory receptors in addition to smell recognition have been conducted.

The olfactory receptor 51E2 (OR51E2) used in the present invention is highly expressed in the prostate, thereby being also called PSGR (Prostate-Specific G-Protein Coupled Receptor). It is known to be involved in the malignancy of prostate cancer, but the function in thyroid and thyroid cancer is unknown.

In one embodiment of the present invention, the ligand of the olfactory receptor OR51E2 may be selected from the group consisting of acetate, nonanoic acid, propionate, beta-ionone, azelaic acid, estriol, epitestosterone, 19-OH AD (19-hydroxyandrost-4-ene-3,17-dione), palmitic acid, androstenedione, D-alanyl-d-alanine, glycylglycine, kojibiose, urea, AFMK (N-acetyl-N-formyl-5-metoxykynurenamine), pelargonidin, hydroxypyruvic acid, bradykinin, 8-hydroxyguanine, imidazolone, 2-pyrrolidinone, 2-ketoglutaric acid, L-glyceric acid, glycine and 13-cis retinoic acid, and preferably acetate may be used.

In one embodiment of the present invention, the diagnostic radionuclide may be selected from the group consisting of carbon-11 (C-11), nitrogen-13 (N-13), oxygen-15 (O-15), fluorine-18 (F-18), phosphorus-32 (P-32), copper-64 (Cu-64), gallium-67 (Ga-67), gallium-68 (Ga-68), rubidium-82 (Rb-82), zirconium-89 (Zr-89), technetium-99m (Tc-99m), indium-111 (In-111), iodine-123 (1-123), iodine-131 (1-131), xenon-133 (Xe-133), thallium-201 (Tl-201) and thorium-229 (Th-229).

The term "radionuclide" as used herein refers to an atom with an unstable atomic nucleus, where a nuclear reaction occurs in a process that the atomic nucleus is changed in a stable direction and in this process, radioactive rays such as alpha rays, beta rays and gamma rays are emitted. In general, alpha rays and beta rays have weak penetrating power, but cause degeneration of substances and, in particular, when they cause degeneration in the DNA of living cells, they have strong properties to kill the cells, thereby being used for killing cancer cells. Gamma rays are electromagnetic waves, and when they encounter a substance while proceeding, they have a high probability of escaping through the chemical bonds of the substance, thereby having high penetration force and being used more for disease diagnosis rather than for killing cancer cells.

In the present invention, as a method of labeling a radionuclide to the ligand of OR51E2, a method known in the art may be used. For example, when an element corresponding to a radionuclide is included in the ligand of OR51E2, it can be prepared so that the ligand itself emits radioactive rays by including the radionuclide upon synthesizing the ligand. In addition, some functional groups of the ligand or some elements of the functional group may be substituted with radionuclides within a range without interfering with the ability of the ligand to bind to OR51E2. Alternatively, the ligand and a radionuclide or a compound containing a radionuclide may be linked through a linker. As the linker, a bifunctional chelating agent (BCA), such as DOTA (1,4,7,10-tetraazacyclododecane-1,4,7,10-tetraacetic acid), DTPA (diethylene triamine pentaacetic acid), DO3A (1,4,7,10-tetraazacyclododecane-1, 4,7-triacetic acid), NOTA (1,4,7-triazacyclononane-1,4,7-triacetic acid), NODAGA (1,4,7-triazacyclononane,1-glutaric acid-4,7-acetic acid) and TETA (1,4,8,11-tetraazacyclotetradecane-N,N',N",N'"-tetraacetic acid), may be used. The linker can form a coordination bond with a metallic radioactive isotope, where among the linkers, DOTA or NOTA is often used for labeling Ga-68, and DOTA is widely used for labeling In-111, Y-90, Lu-177, Ga-68, and the like.

In one embodiment of the present invention, acetate may be used as the ligand of OR51E2, and in this case, it may be used by substituting a carbon element of acetate with carbon-11. The carbon-11 has a half-life of 20 minutes, and since carbon becomes a constituent element of all organic substances, it can be labeled without changing the properties of organic substances.

Another aspect of the present invention provides a method for diagnosing medullary thyroid cancer comprising the following steps:
(a) administering the composition of any one of claims 1 to 4 to an individual suspected of developing medullary thyroid cancer;
(b) detecting a signal of the diagnostic radionuclide of the administered composition; and
(c) comparing the detected signal of the diagnostic radionuclide with that of a normal individual.

In one embodiment of the present invention, the method for diagnosing medullary thyroid cancer further comprises a step of determining that the individual has developed medullary thyroid cancer when the detected signal in step (c) is higher than that of the normal individual.

In the present invention, the olfactory receptor OR51E2 is also expressed in normal parafollicular C-cells, but when medullary thyroid cancer is developed, the number of parafollicular C-cells increases and the signal of the diagnostic radionuclide appears high as compared with the normal individual (individual without developing medullary thyroid cancer), so that it is possible to determine whether or not medullary thyroid cancer is developed.

Another aspect of the present invention provides a method for identifying metastasis of medullary thyroid cancer comprising the following steps:
(a) administering the composition of any one of claims 1 to 4 to an individual suffering from medullary thyroid cancer;
(b) detecting a signal of the diagnostic radionuclide of the administered composition; and
(c) identifying whether or not the signal of the detected diagnostic radionuclide appears in areas other than the thyroid gland.

In the present invention, the olfactory receptor OR51E2 is expressed in parafollicular C-cells of the thyroid gland, and when the medullary thyroid cancer is metastasized, the signal of the diagnostic radionuclide appears at a site other than the thyroid gland by the movement of the parafollicular C-cells, so that it is possible to determine whether or not medullary thyroid cancer has metastasized and the metastasis location by grasping the location where the signal appears.

As a result of identifying the olfactory receptors expressed in the thyroid gland, the present inventors have confirmed that OR51E1 and OR51E2 are specifically expressed in parafollicular C-cells of the thyroid gland, and that OR51E2 migrates into the cells when treated with acetate as the ligand of OR51E2. Thus, the radionuclide-labeled ligand of OR51E2 can migrate into the parafollicular C-cells together with OR51E2, and consequently, the parafollicular C-cells can be identified with the radionuclide.

Another aspect of the present invention provides a pharmaceutical composition for treating medullary thyroid cancer comprising acetate and a therapeutic radionuclide associated therewith as active ingredients.

In the present invention, the active ingredients of the pharmaceutical composition comprise both acetate and a therapeutic radionuclide associated therewith, or radionuclide-labeled acetate.

In the present invention, the therapeutic radionuclide refers to a substance that destroys cancer cells by emitting radioactive rays when it reaches a target such as cancer in a living body. For example, the therapeutic radionuclide may be selected from the group consisting of carbon-11 (C-11), nitrogen-13 (N-13), oxygen-15 (0-15), fluorine-18 (F-18), phosphorus-32 (P-32), copper-64 (Cu-64), gallium-67 (Ga-67), gallium-68 (Ga-68), rubidium-82 (Rb-82), zirconium-89 (Zr-89), Technetium-99m (Tc-99m), Indium-111 (In-111), Iodine-123 (1-123), Iodine-131 (1-131), xenon-133 (Xe-133), Thallium-201 (Tl-201), thorium-229 (Th-229), yttrium-90 (Y-90), holmium-166 (Ho-166) and rhenium-188 (Re-188). Preferably, iodine-123 (1-123) or iodine-131 (1-131) may be used, and most preferably, iodine-131 (1-131) is used.

The present inventors have confirmed that OR51E1 and OR51E2 are specifically expressed in parafollicular C-cells, which are the origin cells of medullary thyroid cancer, and that OR51E2 migrates into cells when treated with acetate as the ligand of OR51E2. Therefore, the acetate associated with a therapeutic radionuclide or substituted with the radionuclide for the carbon element can move into parafollicular C-cells together with OR51E2, and can kill cancer cells by emitting radioactive rays within the cells. In particular, since radionuclides with known therapeutic effects are used, it is possible to increase the killing effect of medullary thyroid cancer cells. As a method of binding acetate with a therapeutic radionuclide, the same method as the labeling method of the diagnostic radionuclide may be used.

Therefore, the present invention provides a method for treating medullary thyroid cancer comprising a step of administering the pharmaceutical composition to an individual in need of treatment for medullary thyroid cancer.

The pharmaceutical composition of the present invention may further comprise suitable carriers, excipients and diluents conventionally used in the manufacture of pharmaceuticals in addition to the above active ingredients.

The pharmaceutical compositions according to the present invention may be formulated in the form of oral dosage forms such as powders, granules, tablets, capsules, suspensions, emulsions, syrups and aerosols, external preparations, suppositories, and sterile injectable solutions, respectively, according to conventional methods and used. The carriers, excipients and diluents that may be included in the composition of the present invention may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oils.

In the case of formulation, it is prepared using diluents or excipients such as fillers, extenders, binders, wetting agents, disintegrants and surfactants that are usually used. Solid preparations for oral administration include tablets, pills, powders, granules, capsules, and the like, and these solid preparations are prepared by mixing at least one excipient, for example, starch, calcium carbonate, sucrose or lactose, gelatin, and the like with the composition of the present invention. In addition to simple excipients, lubricants such as magnesium stearate and talc are also used. Suspensions, oral liquids, emulsions, syrups, and the like correspond to liquid preparations for oral use, where various excipients, for example, wetting agents, sweetening agents, fragrances, preservatives and the like may be included in addition to water and liquid paraffin, which are commonly used simple diluents. Formulations for parenteral administration include sterilized aqueous solutions, non-aqueous solvents, suspensions, emulsions, lyophilized formulations, and suppositories. As the non-aqueous solvents and suspending agents, propylene glycol, polyethylene glycol, vegetable oils such as olive oil, injectable esters such as ethyl oleate, and the like may be used. As a base for suppositories, witepsol, macrogol, tween 61, cacao butter, laurin butter, glycerol gelatin, and the like may be used.

The composition of the present invention may be administered orally or parenterally, any parenteral administration method may be used, systemic administration or local administration may be possible, but systemic administration is more preferable, and intravenous administration is most preferable.

The dosage amount and time of administration of the pharmaceutical composition according to the present invention may vary depending on the age, sex, type of disease, state, weight, administration route and administration number of administration subjects, and the drug form. The daily dosage amount is about 0.00001 to 1000 mg/kg, and preferably 0.0001 to 100 mg/kg. The dosage amount may be appropriately increased or decreased depending on the type of disease, the degree of cancer progression, the administration route, sex, age, weight, and the like.

### Advantageous Effects

The composition for targeting medullary thyroid cancer of the present invention is internalized into parafollicular C-cells by the olfactory receptor OR51E2 expressed in the parafollicular C-cells of the thyroid gland, so that it can be advantageously used for diagnosing parafollicular C-cell-derived medullary thyroid cancer and identifying whether or not parafollicular C-cell-derived medullary thyroid cancer metastases are. And the pharmaceutical composition for treating medullary thyroid cancer is internalized into cancer cells by the binding of acetate and the olfactory receptor OR51E2, so that medullary thyroid cancer can be treated.

### Description of Drawings

Figure 1 shows the results of staining normal thyroid cells and medullary thyroid cancer cells with an olfactory marker protein (OMP) and calcitonin.
Figure 2 shows the results of staining medullary thyroid cancer cell lines MZ-CRC1 and TT, undifferentiated thyroid cancer cell lines FRO and SW1736, a papillary thyroid cancer cell line TPC-1, and a follicular thyroid cancer cell line FTC-133 with an olfactory marker protein antibody.
Figure 3A shows the results of identifying the expression of OR51E1 (olfactory receptor 51E1) and OR51E2 in various thyroid cancer cell lines.
Figure 3B shows the results of staining the thyroid medullary cancer cell lines MZ-CRC1 and TT with OR51E1 antibody.
Figure 3C shows the results of staining the thyroid medullary cancer cell lines MZ-CRC1 and TT with OR51E2 antibody.
Figure 4A shows the results of identifying the phosphorylation level of p42/44 after treating the parafollicular C-cell line with acetate, nonanoic acid, propionate and azelaic acid which are the olfactory receptor ligands.
Figure 4B shows the results of identifying the phosphorylation level of CREB and p42/44 after treating the parafollicular C-cell line with acetate.
Figure 4C shows the results of identifying the level of cAMP.
Figure 5A shows the results of analyzing the degree of cell proliferation with bromodeoxyuridine after treating the parafollicular C-cell line with acetate.
Figure 5B is the results of identifying the intracellular expression level of calcitonin.
Figure 5C shows the results of identifying the release level of calcitonin by inhibiting the expression of OR51E2 with siRNA, followed by acetate treatment.
Figure 6 shows the results of staining thyroid tissues of mice with the olfactory marker protein (OMP), calcitonin and OR51E2 antibody, respectively.
Figure 7A shows the results of staining thyroid tissues of a transgenic mouse, in which the OR51E2 gene is replaced with a green fluorescent protein (GFP), with an antibody.
Figure 7B shows the results of measuring the calcitonin level in serum after administration of acetate to a wild-type mouse and the transgenic mouse.
Figure 8A shows the results of measuring the degree of positron emission after treating various thyroid cancer cell lines with ¹¹C-acetate.
Figure 8B is the results of measuring CPM (counter per minute) after treating various thyroid cancer cell lines with ¹¹C-acetate.
Figure 8C is the results of measuring CPM by introducing siOR51E2 into the medullary thyroid cancer cell line, followed by ¹¹C-acetate treatment.
Figure 8D shows the results of measuring CPM after treating OR51E2 knockout mice with ¹¹C-acetate.
Figure 9A shows the results of measuring CPM by first treating MZ-CRC-1 cell line, which is a medullary thyroid cancer cell line, with cold acetate, followed by competitive treatment with ¹¹C-acetate.
Figure 9B shows the results of measuring CPM by first treating TT cell line, which is a medullary thyroid cancer cell line, with cold acetate, followed by competitive treatment with ¹¹C-acetate.
Figure 9C shows the results of measuring CPM by first treating MZ-CRC-1 cell line, which is a medullary thyroid cancer cell line, with ¹¹C-acetate, followed by competitive treatment with cold acetate.
Figure 9D shows the results of measuring CPM by first treating TT cell line, which is a medullary thyroid cancer cell line, with ¹¹C-acetate, followed by competitive treatment with cold acetate.
Figure 10 shows the results of staining the cells with an OR51E2 antibody after treating the MZ-CRC-1 cell line with acetate.
Figure 11A shows the results of performing positron emission tomography (PET) after administering ¹¹C-acetate to a nude mouse in which a tumor has been formed by administering the MZ-CRC-1 cell line.
Figure 11B shows the results of measuring the calcitonin level in the serum of the nude mouse.
Figure 12 shows the results of performing PET after administering ¹¹C-acetate to a patient with medullary thyroid cancer.

### Mode for Invention

Hereinafter, one or more embodiments will be described in more detail through examples. However, these examples are intended to illustratively explain one or more embodiments and the scope of the present invention is not limited to these examples.

### Example 1: Expression identification of olfactory marker protein

### 1-1. Thyroid tissue and thyroid cancer cell line staining

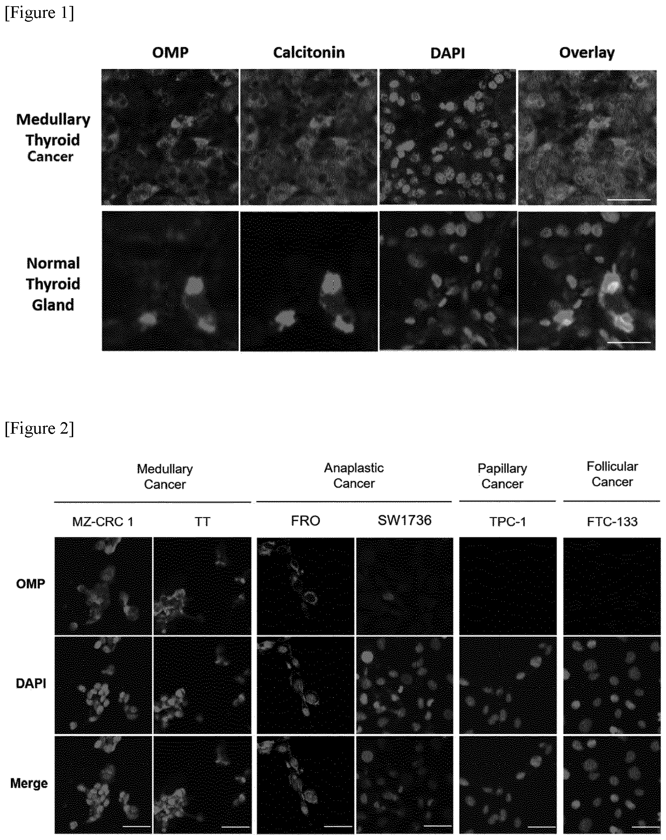

In order to identify whether an olfactory receptor is expressed in the thyroid gland, the expression of the olfactory marker protein (hereinafter, referred to as OMP) was identified. As a result of staining normal thyroid gland tissues and medullary thyroid cancer (MTC) cells with an OMP antibody (rabbit anti-OMP; Santa Cruz, CA, USA), it was identified that the OMP was expressed in the thyroid gland, and it could be seen that the OMP was expressed only in parafollicular C-cells among cells constituting the thyroid gland (Figure 1). Medullary thyroid cancer is a malignant thyroid tumor originating from parafollicular C-cells, where calcitonin is a marker protein for parafollicular C-cells.

### 1-2. Thyroid cancer cell line staining

MZ-CRC1 and TT as medullary thyroid cancer cell lines, FRO and SW1736 as undifferentiated thyroid cancer cell lines, TPC-1 as a papillary thyroid cancer cell line, FTC-133 as a follicular thyroid cancer cell line were stained with an OMP antibody.

As a result, it could be identified that OMPs were highly expressed in MZ-CRC1 and TT cell lines as medullary thyroid cancer cell lines derived from parafollicular C-cells among various thyroid cancer cell lines (Figure 2).

### 1-3. Identification of types of olfactory receptors

To identify the types of olfactory receptors expressed in thyroid cancer cell lines, RNA was isolated and then cDNA was synthesized to perform real-time polymerase chain reaction (RT-PCR) according to a method known in the art.

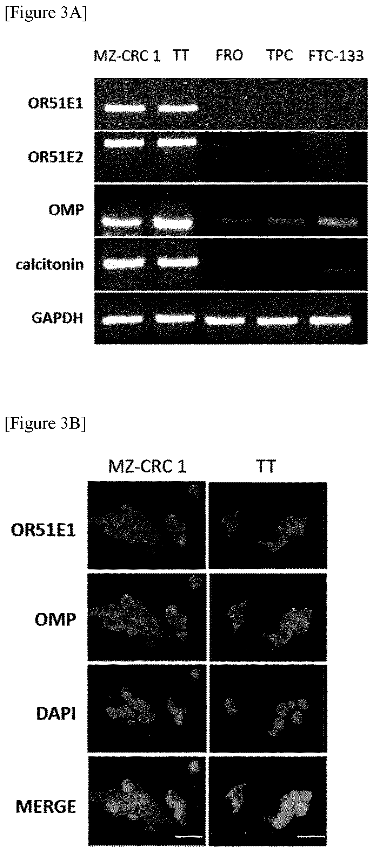

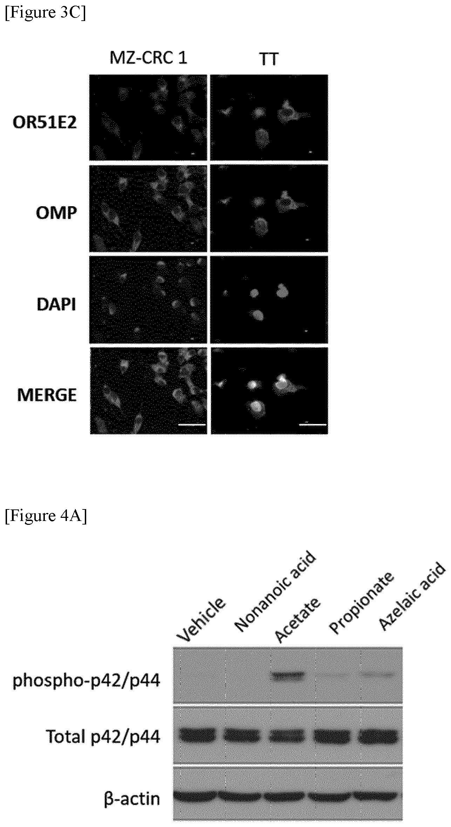

As a result, it could be identified that OR51E1 (olfactory receptor 51E1) and OR51E2 were highly expressed in MZ-CRC1 and TT cell lines as medullary thyroid cancer cell lines, but rarely expressed in other thyroid cancer cell lines (A in Figure 3). In addition, as a result of staining the MZ-CRC1 and TT cell lines with OR51E1 and OR51E2 antibodies, respectively, it could be seen that the expression of the olfactory receptor almost coincided with the expression pattern of OMP (B and C in Figure 3).

### Example 2: Identification of influences of olfactory receptor ligands

### 2-1. Treatment of olfactory receptor ligands on parafollicular C-cells

Since OR51E1 and OR51E2 were highly expressed in the medullary thyroid cancer cell lines, MZ-CRC1 and TT cell lines as parafollicular C-cell lines were treated with acetate, nonanoic acid, propionate and azelaic acid as olfactory receptor ligands by 100 µM each to identify their influences.

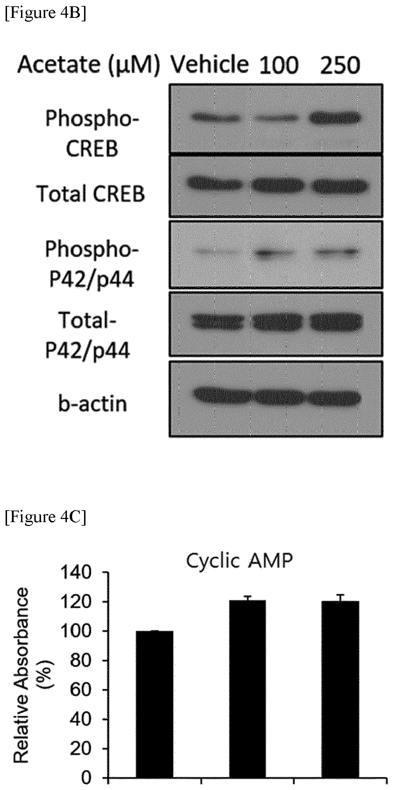

As a result of the experiment, it could be seen that the level of phosphorylation of p42/44 MAPK (mitogen-activated protein kinase) was increased by acetate among various ligands (A in Figure 4). In addition, the phosphorylation levels of p42/44 MAPK and CREB (cAMP response element-binding protein) increased in proportion to the acetate treatment concentration (B in Figure 4), and as the phosphorylation level of CREB increased, the level of intracellular cAMP also increased. (C in Figure 4).

Thereafter, the parafollicular C-cells were treated with acetate at each concentration and cultured, and then using a bromodeoxyuridine (BrdU) cell proliferation assay kit (Millipore, Massachusetts), the degree of BrdU binding was analyzed according to the manufacturer's protocol.

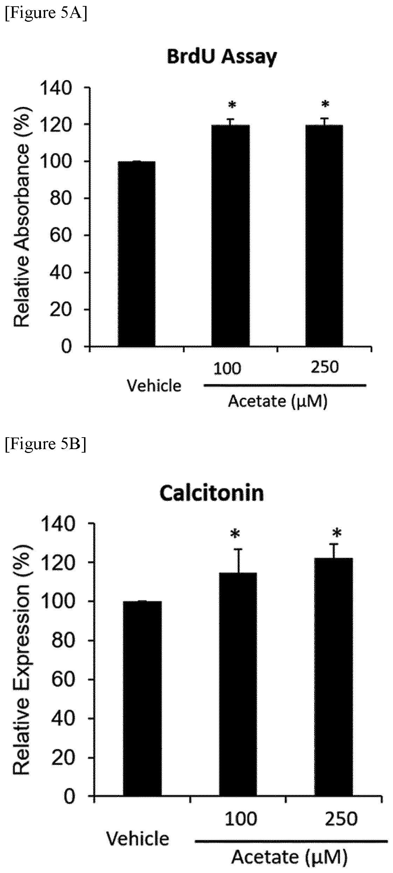

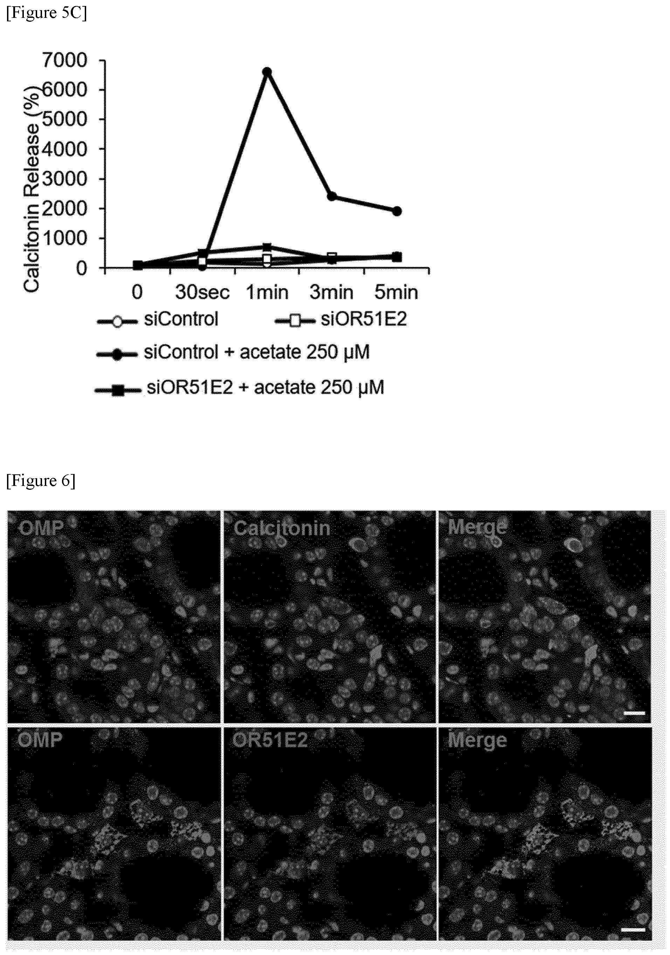

As a result, it could be seen that the cell proliferation was increased by identifying the increase of the BrdU binding level by the acetate treatment (A in Figure 5). In addition, the intracellular expression level of calcitonin was increased by the acetate treatment (B in Figure 5), and when the expression of OR51E2 was suppressed with siRNA, the release of calcitonin was not increased even with the acetate treatment (C in Figure 5).

### 2-2. Thyroid tissue staining in mouse

The thyroid gland was isolated from an 8-week-old female C57BL/6 mouse to prepare tissue sections, and OR51E2, calcitonin, and OMP were stained with the respective antibodies. As a result, it could be seen that OR51E2, OMP, and calcitonin were co-localized in the thyroid tissue (Figure 6).

### 2-3. OR51E2 knockout mice

C57/BL6 mice in which a green fluorescent protein (GFP) was inserted into the OR51E2 gene loci were purchased (Jackson Laboratory, USA) and reared under sterile conditions. Through a week of acclimatization, the mice were each sacrificed 30 minutes after injecting 100 µl of 99% acetate thereto to separate the thyroid gland and serum. The thyroid gland was stained with calcitonin and a GFP antibody, respectively, and the calcitonin concentration was measured in the separated serum.

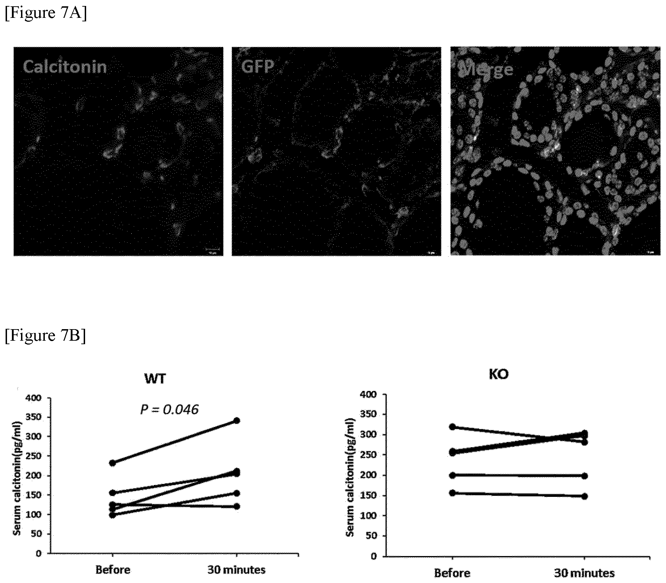

As a result of thyroid gland staining, it could be identified that the calcitonin and GFP were located at the same site (A in Figure 7), and it could be seen that in the case of wild-type mice, the level of calcitonin in the serum was increased by acetate, but in OR51E2 knockout mice, the level of calcitonin was maintained almost constant (B in Figure 7).

### Example 3: Identification of acetate influences on olfactory receptor OR51E2

### 3-1. ¹¹C-acetate treatment

Thyroid cancer cell lines having different origins were treated with ¹¹C-acetate labeled with a radioactive isotope (20 µCi), and the degree of positron emission was identified. A control group was treated with F-18-fluorodeoxyglucose as the most commonly used radiopharmaceutical.

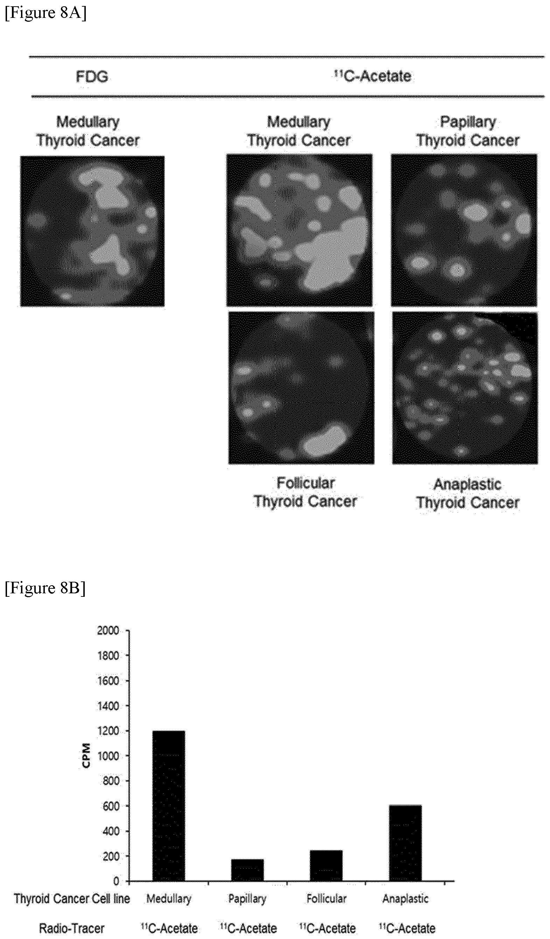

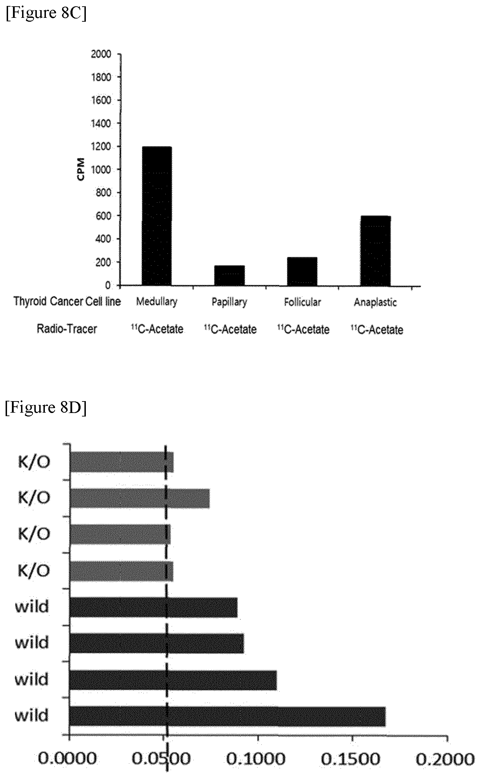

As a result, it was shown that the level of positron emission was high in the medullary thyroid cancer cell line, whereby it could be seen that acetate was bound to OR51E2 of the parafollicular C-cells (A in Figure 8), and it was shown that the result of measuring CPM (counter per minute) also was the highest in the medullary thyroid cancer cell line (B in Figure 8).

In the case of suppressing the expression of OR51E2 by treating the thyroid medullary cancer cell line with siOR51E2, CPM did not increase even with the ¹¹C-acetate treatment (C in Figure 8), and in the case of OR51E2 knockout mice, CPM did not increase even with the ¹¹C-acetate treatment as compared with wild-type mice (D in Figure 8).

### 3-2. Acetate binding competition experiment

MZ-CRC-1 and TT cell lines as medullary thyroid cancer cell lines were treated with 100 µM cold acetate (acetate labeled with no radioactive isotope), and then treated with ¹¹C-acetate at concentrations of 2, 5, 10, 20, 40 and 60 µCi to measure CPM. In addition, MZ-CRC-1 and TT cell lines were treated with ¹¹C-acetate (20 µCi), and then treated with cold acetate at concentrations of 0, 20, 40, 80, 150 and 300 µM to measure CPM.

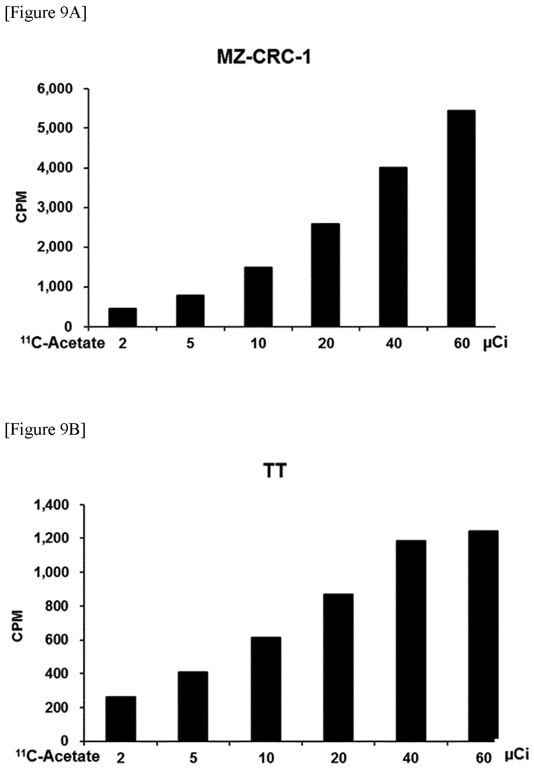

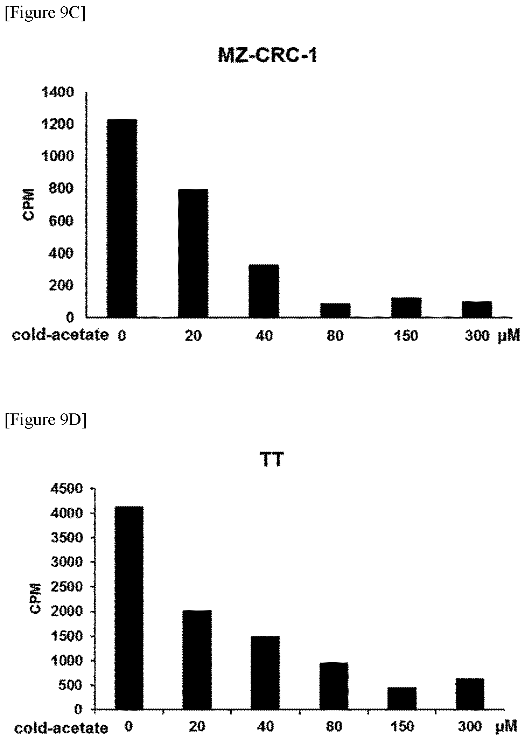

In the case of the first cold acetate treatment and ¹¹C-acetate treatment, CPM increased as the treatment concentration increased (A and B in Figure 9). Conversely, in the case of the ¹¹C-acetate treatment and then cold acetate treatment, CPM decreased as the treatment concentration increased (C and D in Figure 9). This result means that cold acetate and ¹¹C-acetate bind to OR51E2 competitively with each other.

### 3-3. Use of acetate to diagnose thyroid cancer

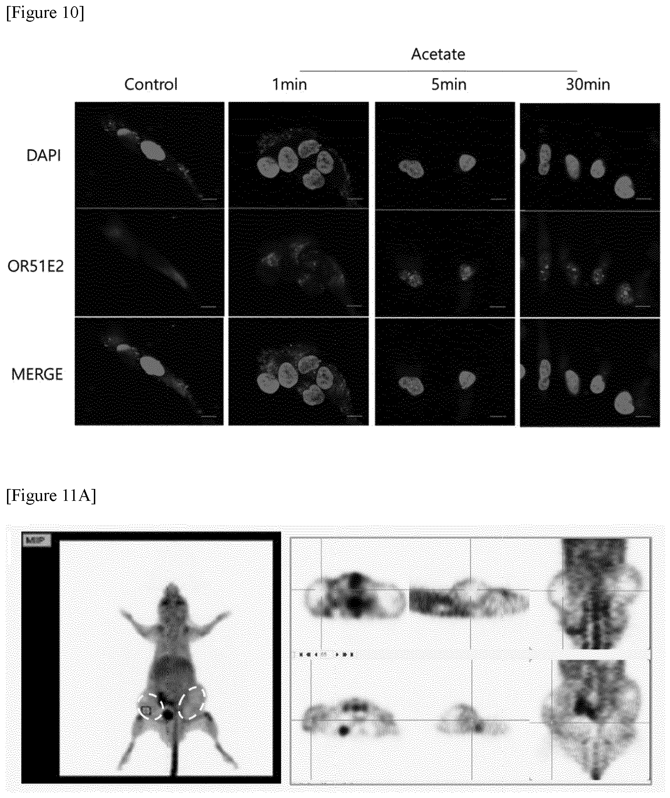

To identify whether the binding of acetate and OR51E2 can be utilized in the diagnosis of thyroid cancer, the MZ-CRC-1 cell line (parafollicular C-cells) was treated with 100 µM of acetate, and the cells were stained after 1, 5 and 30 minutes to identify OR51E2. As a result, it could be identified that OR51E2 migrated into the cells by the acetate treatment (Figure 10).

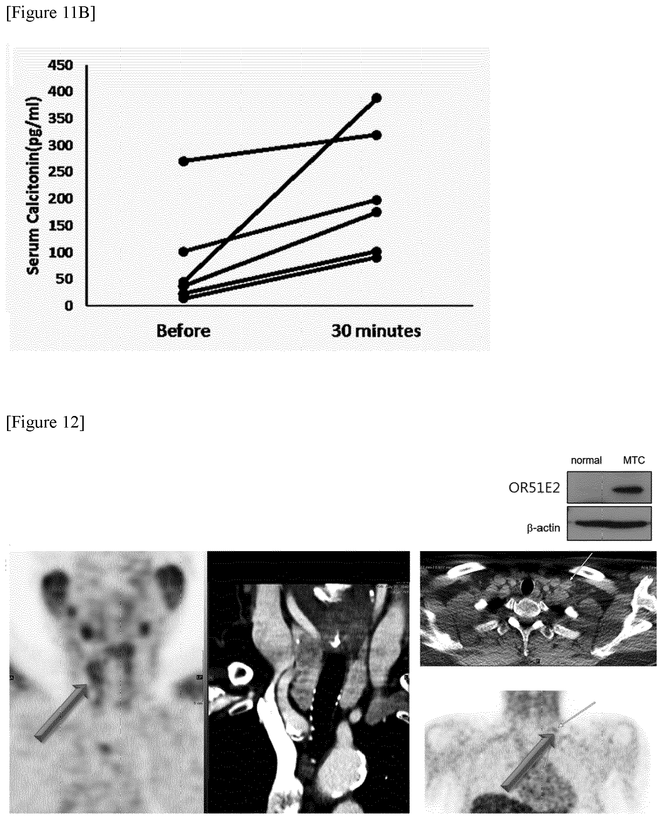

Nude mice (BALB/c) were administered with the MZ-CRC-1 cell line (4x10⁶ cells/mouse) to induce tumorigenesis, and administered with ¹¹C-acetate, followed by performing positron emission tomography (PET). As a result, it could be seen that ¹¹C-acetate was accumulated in the tumor-producing sites by the MZ-CRC-1 cell line (A in Figure 11), and it could be identified that the calcitonin concentration in the serum was increased by the administration of ¹¹C-acetate (B in Figure 11).

Through the results so far, it was identified that the ligand of the olfactory receptor OR51E2 was acetate, and it was identified that since OR51E2 migrated into the cells by the binding of OR51E2 and acetate, acetate could be used for diagnosis of medullary thyroid cancer.

## Claims

1. A composition for targeting medullary thyroid cancer, comprising a diagnostic radionuclide-labeled ligand of an olfactory receptor OR51E2.

2. The composition for targeting medullary thyroid cancer according to claim 1, wherein the medullary thyroid cancer is derived from parafollicular C-cells.

3. The composition for targeting medullary thyroid cancer according to claim 1, wherein the diagnostic radionuclide is selected from the group consisting of carbon-11 (C-11), nitrogen-13 (N-13), oxygen-15 (O-15), fluorine-18 (F-18), phosphorus-32 (P-32), copper-64 (Cu-64), gallium-67 (Ga-67), gallium-68 (Ga-68), rubidium-82 (Rb-82), zirconium-89 (Zr-89), technetium-99m (Tc-99m), indium-111 (In-111), iodine-123 (1-123), iodine-131 (I-131), xenon-133 (Xe-133), thallium-201 (Tl-201) and thorium-229 (Th-229).

4. The composition for targeting medullary thyroid cancer according to claim 1, wherein the ligand of the olfactory receptor OR51E2 is acetate, propionate, nonanoic acid, beta-ionone, azelaic acid, estriol, epitestosterone, 19-OH AD (19-hydroxyandrost-4-ene-3,17-dione), palmitic acid, androstenedione, D-alanyl-d-alanine, glycylglycine, kojibiose, urea, AFMK (N-acetyl-N-formyl-5-metoxykynurenamine), pelargonidin, hydroxypyruvic acid, bradykinin, 8-hydroxyguanine, imidazolone, 2-pyrrolidinone, 2-ketoglutaric acid, L-glyceric acid, glycine or 13-cis retinoic acid.

5. A method for diagnosing medullary thyroid cancer, comprising steps of:
(a) administering the composition of any one of claims 1 to 4 to an individual suspected of developing medullary thyroid cancer;
(b) detecting a signal of the diagnostic radionuclide of the administered composition; and
(c) comparing the detected signal of the diagnostic radionuclide with that of a normal individual.

6. The method for diagnosing medullary thyroid cancer according to claim 5, wherein the method for diagnosing medullary thyroid cancer further comprises a step of determining that the individual has developed medullary thyroid cancer when the detected signal in step (c) is higher than that of the normal individual.

7. A method for identifying metastasis of medullary thyroid cancer, comprising steps of:
(a) administering the composition of any one of claims 1 to 4 to an individual suffering from medullary thyroid cancer;
(b) detecting a signal of the diagnostic radionuclide of the administered composition; and
(c) identifying whether or not the signal of the detected diagnostic radionuclide appears in areas other than the thyroid gland.

8. A pharmaceutical composition for treating medullary thyroid cancer comprising acetate and a therapeutic radionuclide associated therewith as active ingredients.

9. The pharmaceutical composition for treating medullary thyroid cancer according to claim 8, wherein the therapeutic radionuclide is selected from the group consisting of carbon-11 (C-11), nitrogen-13 (N-13), oxygen-15 (0-15), fluorine-18 (F-18), phosphorus-32 (P-32), copper-64 (Cu-64), gallium-67 (Ga-67), gallium-68 (Ga-68), rubidium-82 (Rb-82), zirconium-89 (Zr-89)), technetium-99m (Tc-99m), indium-111 (In-111), iodine-123 (I-123), iodine-131 (I-131), xenon-133 (Xe-133), thallium-201 (Tl-201), thorium-229 (Th-229), yttrium-90 (Y-90), holmium-166 (Ho-166) and rhenium-188 (Re-188).

10. A method for treating medullary thyroid cancer comprising a step of administering the pharmaceutical composition for treating medullary thyroid cancer of claim 8 or 9 to an individual in need of treatment.
